# EUROPEAN PATENT SPECIFICATION

(11) **EP 0 511 019 B1**
(45) Date of publication and mention of the grant of the patent: **23.06.1999**
(21) Application number: 92303748.5
(22) Date of filing: 24.04.1992
(51) Int. Cl.: C07C 227/02, C07C 227/16, C07C 229/52

(54) **Method of producing keto acids**
Verfahren zur Herstellung von Ketosäuren
Procédé pour la fabrication d'acides cétoniques

(30) Priority: 25.04.1991 JP 9590191; 11.03.1992 JP 5288992
(43) Date of publication of application: 28.10.1992
(60) Divisional application: 98106734.1
(73) Proprietor: MITSUI CHEMICALS, INC., Tokyo (JP)
(72) Inventor: Kondo, Masahiro, c/o Mitsui Petrochem. Ind. Ltd., Kuga-gun, Yamaguchi (JP); Tanaka, Michio, c/o Mitsui Petrochem. Ind. Ltd., Kuga-gun, Yamaguchi (JP); Sakamoto, Naoya, c/o Mitsui Petrochem. Ind. Ltd., Kuga-gun, Yamaguchi (JP); Ooyoshi, Hajime, c/o Mitsui Petrochem. Ind. Ltd., Kuga-gun, Yamaguchi (JP)
(74) Representative: Cresswell, Thomas Anthony

(56) References cited:
- DE-A- 3 407 369
- DE-A- 3 415 331
- DE-C- 85 931
- GB-A- 1 182 743
- GB-A- 1 306 263
- CHEMICAL ABSTRACTS, vol. 108, 1988, Columbus, Ohio, US; abstract no. 23350b, & JP-A-62 070 350 (KANZAKI PAPER MFG CO LTD)

## Description

This invention relates to a method of producing keto acids.

Keto acids are useful intermediates for the production of fluoran compounds used as dyestuffs in pressure- or heat-sensitive recording.

Keto acids have hitherto been produced by the reaction of an N,N-dialkyl-m-aminophenol with phthalic anhydride in a molar ratio of phthalic anhydride to N,N-dialkyl-m-aminophenol of 0.5-2. Both compounds are dissolved in an inactive organic solvent such as toluene, xylene or tetrahydrofuran at a temperature of 80-150°C. However, the method provides a considerable amount of Rhodamines known as red dyes by the reaction of the resultant keto acids with the N,N-dialkyl-m-aminophenol. This reduces the yield of the keto acids, and makes it difficult to obtain high purity keto acids.

To solve the above problem, there has been proposed a method in which an aqueous solution of an alkali metal hydroxide such as sodium hydroxide is added to the resultant reaction mixture, the reaction mixture is heated to decompose by-produced Rhodamines to alkali metal salts of the keto acid, the alkali metal salt of the keto acid is crystallized and then dissolved again in water, and than the salt is neutralized in water to recover the keto acid, as disclosed in JP-A-62-70350. However, this method needs many steps, and, in addition, produces a large amount of neutralization waste water.

DE-A-3,407,369 discloses in Preparation Example 1 a reaction between m-N-n-propylmethylaminophenol and phthalic acid in toluene. This reaction is purely carried out in solution.

DE-A-8,5931 discloses in Example II the condensation of N,N-diethyl-m-aminophenol with phthalic anhydride in the presence of 4.54 parts by weight of toluene per part by weight of the N,N-diethyl-m-aminophenol. It is observed in the specification that the precipitation of crystals can be used as an indicator of completion of reaction. DE-A-8,5931 is referenced in GB-A-1,306,263 as describing the synthesis of a starting material for fluoran compound synthesis.

GB-A-1,182,743, which is also directed to fluoran compound synthesis, also discloses condensation of N,N-diethyl-m-aminophenol with phthalic anhydride in toluene to provide a starting material, the toluene being present in 3.93 parts by weight per part by weight of N,N-diethyl-m-aminophenol. The condensation product is disclosed as being gradually crystallised from solution.

The present invention seeks to provide a method of producing keto acids of high purity in high yield by the reaction of an N,N-dialkyl-m-aminophenol with phthalic anhydride while suppressing undesirable by-production of Rhodamines.

The present invention provides a method of producing a keto acid of the general formula 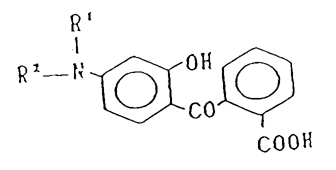 wherein R¹ and R² independently represent alkyl of 1-6 carbons or cycloalkyl of 4-8 carbons, which comprises reacting a m-aminophenol of the general formula 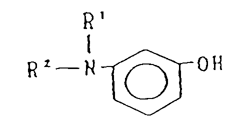 wherein R¹ and R² are as herein defined with phthalic anhydride in the Presence of an organic solvent, the organic solvent being present in an amount of 0.5-3 parts by weight per one part by weight of the m-aminophenol with the effect that the resultant keto acid is deposited in the solvent so that the reaction is effected in a slurry.

The m-aminophenol wherein both R¹ and R² are alkyls of 1-6 carbons used in the invention includes, for example, N,N-dimethyl-m-aminophenol, N,N,-diethyl-m-aminophenol, N,N-di-n-propyl-m-aminophenol, N,N-di-isopropyl-m-aminophenol, N,N-di-n-butyl-m-aminophenol, N-methyl-N-ethyl-m-aminophenol, N-ethyl-N-isopropyl-m-aminophenol, N-ethyl-N-n-butyl-m-aminophenol and N-ethyl-N-isoamyl-m-aminophenol. The m-aminophenol wherein one of R¹ and R² is cycloalkyl may be exemplified by N-ethyl-N-cyclohexyl-m-aminophenol.

For the reaction of the m-aminophenol derivative as above mentioned with phthalic anhydride, the latter is usually used in an amount of 0.7-2 moles per mole of the m-aminophenol derivative. The reaction is effected in a controlled amount of an organic solvent so that the resultant keto acid deposits in the solvent thereby to form a slurry, and the reaction is allowed to proceed in such a slurry. Strictly speaking, the amount of the solvent used is determined so that the resultant keto acid deposits in the solvent and thus the reaction proceeds in a slurry of the keto acid in the solvent. However, the amount of the solvent is usually in the range of 0.5-3 parts by weight in relation to one part by weight of the m-aminophenol derivative used.

The organic solvent used includes, for example, an aromatic hydrocarbon of 6-10 carbons such as benzene, toluene or xylene, an aliphatic hydrocarbon of 8-12 carbons such as octane, isooctane or decane, a halogenated hydrocarbon of 2-8 carbons, aliphatic, cycloaliphatic or aromatic, such as perchlene or chlorobenzene, ethers such as tetrahydrofuran, dibutyl ether or diphenyl ether, among which are especially preferred aromatic hydrocarbons or ethers.

By way of example, when the reaction of N,N-di-n-butyl-m-aminophenol with phthalic anhydride is carried out in an aromatic hydrocarbon such as benzene, toluene or xylene, the preferred amount of the solvent is in the range of 0.5-2 parts by weight in relation to one part by weight of the N,N-di-n-butyl-m-aminophenol.

The reaction is effected at an elevated temperature, preferably at a temperature of 60-120°C for a period of 4-40 hours, although the reaction temperature and time are not critical. After the reaction, the reaction mixture is cooled usually to normal temperature, preferably to a temperarture of 0-35°C, most preferably to 10-30°C, depending upon the solvent used, or a poor solvent such as a saturated hydrocarbon is added to the reaction mixture, to effect primary crystallization of the resultent keto acid.

The resultant crude crystals may then be dissolved under heating in an aliphatic alcohol of 1-4 carbons and then secondary crystallization is effected. The secondary crystallization may be effected at the same temperature range as the primary crystallization. The secondary crystallization enables a high purity keto acid which contains substantially no Rhodamine impurities to be obtained.

There may be used as the alcohol for the secondary crystallization solvent, for example, methanol, ethanol, propanols such as isopropanol or butanols such as n-butanol. There may also be used a mixture of the alcohol with water, or a mixture of the alcohol with a hydrocarbon solvent, preferably an aromatic hydrocarbon of 6-10 carbons such as toluene or xylene, or an aliphatic hydrocarbon of 5-10 carbons such as pentane, hexane or heptane.

The crude crystals may be dissolved in the alcohol under elevated pressure, usually under a pressure of several atmospheric pressures, and then the solution may be cooled to effect the secondary crystallization.

Further according to the invention, after the reaction, an aliphatic alcohol of 1-4 carbons may be added to the reaction mixture and then primary crystallization may be effected. The addition of the aliphatic alcohol to the reaction mixture enables selective dissolution of Rhodamines so that the slurry is kept in a good state from which the resultant crystals can easily be collected by filtration.

As set forth above, the reaction of the m-aminophenol derivative with phthalic anhydride is carried out in a controlled amount of an organic solvent to allow the resultant keto acid to deposit in the solvent and the reaction to proceed in a slurry according to the invention. Thus, the by-production of undesirable Rhodamines is effectively suppressed to improve the selectivity of the reaction to the keto acid. In addition, there is obtained a high purity keto acid which contains substantially no Rhodamine impurities by effecting secondary crystallization of the resultant keto acid out of the alcohol.

Further according to the invention, the alcohol may be recovered from the the secondary crystallization mother liquor, and if necessary, the alcohol is completely removed, to provide a residual solid which contains the keto acid. The solid is then dissolved in an inactive organic solvent which can be used as a reaction solvent, the thus obtained solution may be added to the reaction mixture, and the mixture is then cooled to effect primary crystallization. This results in a remarkable improvement in the yield of the keto acid.

The amount of undesirable Rhodamines by-produced in the reaction is small according to the invention, and thus the ratio of the Rhodamines to the keto acid in the mother liquor after the secondary crystallization is very small. Therefore, according to the invention, the solvent in the mother liquor may be exchanged with an organic solvent which can be used as a reaction solvent, and the resultant solution containing the keto acid can be advantageously used for primary crystallization together with the reaction mixture, thereby increasing the yield of the keto acid.

The invention will now be described in more detail with in the following Examples.

### Example 1

165 g (1.0 sole) of N,N-diethyl-m-aminophenol, 170.3 g (1.15 mole) of phthalic anhydride and 206 g of xylene were placed in a reactor, and stirred for 7 hours at 115°C while allowing the resultant keto acid (4-N,N-diethylamino-2-hydroxy-2'-carboxybenzophenone) to deposit in the reaction mixture and the reaction to proceed in a slurry.

After the reaction, 247 g of xylene were added to the reaction mixture, and the mixture was cooled gradually to 20°C so that the keto acid crystalized out. The crystals were collected by filtration and washed with 577 g of n-butanol to provide 303.9 g of crude crystals.

1486 g of n-butanol was added to the crystals and heated to dissolve the crystals therein, end then the mixture was gradually cooled to 20°C. The resultant crystals were collected by filtration and dried to provide 291.9 g of high purity keto acid (4-N,N-diethylamino-2-hydroxy-2'-carboxybenzophenone). The amount of Rhodamines in the keto acid was found to be not more than 0.1% by liquid chromatographic analysis. The yield was 93.1 mol %.

### Example 2

After the reaction, n-butanol was added to the reaction mixture in place of xylene, and otherwise in the same manner as in Example 1, crude crystals were obtained.

1486 g of n-butanol was added to 303.6 g of the crystals and heated to dissolve the crystals therein, and then the mixture was gradually cooled to 20°C. The resultant pale yellow crystals were collected by filtration and dried to provide 291.5 g of high purity keto acid (4-N,N-diethylamino-2-hydroxy-2'-carboxybenzophenone).

No Rhodamines were detected in the keto acid by liquid chromatographic analysis. The yield was 93.5 mol %.

### Example 3

The reaction was effected in toluene in place of xylene, after the reaction methanol was added in place of xylene to the reaction mixture, and the mixture was cooled gradually to 20°C so that the keto acid crystalized out and the crystals were washed with methanol, and otherwise in the same manner as in Example 1.

913 g of methanol was added to 304.5 g of the crude crystals and heated to 113°C under a pressure of 3 Kg/cm² to completely dissolve the crystals therein, and then the mixture was gradually cooled to 20°C. The resultant pale yellow crystals were collected by filtration and dried to provide 295.3 g of high purity keto acid (4-N,N-diethylamino-2-hydroxy-2'-carboxybenzophenone).

No Rhodamines were detected in the keto acid by liquid chromatographic analysis. The yield was 94.3 mol %.

### Example 4

221 g (1.0 mole) of N,N-di-n-butyl-m-aminophenol, 177.7 g (1.2 mole) of phthalic anhydride and 220 ml of xylene were placed in a reactor, and stirred for 7 hours at 100°C while allowing the resultant keto acid (4-N,N-di-n-butylamino-2-hydroxy-2'-carboxybenzophenone) to deposit In the reaction mixture and the reaction to proceed in a slurry.

After the reaction, 220 ml of xylene were added to the reaction mixture, and the mixture was gradually cooled to 20°C so that the keto acid crystalized out. The crystals were collected by filtration and washed with 80 ml of xylene twice.

320 g of the resultant crude crystals was added to 1250 g of methanol. The mixture was heated to dissolve the crystals in the methanol, and then the mixture was cooled to 20°C gradually to effect recrystallization of the keto acid. The resultant crystals were collected by filtration and washed with 100 g of cold methanol twice followed by drying the crystals to provide 255 g of high purity keto acid (4-N,N-di-n-butylamino-2-hydroxy-2'-carboxybenzophenone).

No Rhodamines were detected in the keto acid by liquid chromatographic analysis. The yield was 69.0 mol %.

### Example 5

The reaction was effected in toluene in place of xylene, and otherwise in the same manner as in Example 4 to provide 258 g of high purity keto acid (4-N,N-di-n-butylamino-2-hydroxy-2'-carboxybenzophenone).

No Rhodamines were detected in the keto acid by liquid chromatographic analysis. The yield was 69.8 mol %.

### Example 6

The crystals of keto acid (4-N,N-di-n-butylamino)-2-hydroxy-2'-carboxybenzophenone) obtained in Example 4 were recrystallized, collected by filtration, and washed with cold methanol. The mother liquor was distilled to recover methanol.

220 ml of xylene was added to the distillation bottom containing the keto acid to dissolve the keto acid therein.

The resultant solution was added to the same reaction mixture as obtained in Example 1 and then primary crystallization was effected, followed by working in the same manner as in Example 1. There were obtained 299 g of pale yellow crystals of high purity keto acid (4-N,N-di-n-butylamino-2-hydroxy-2'-carboxybenzophenone).

No Rhodamines were detected in the keto acid by liquid chromatographic analysis. The yield was 81 mol % based on the N,N-di-n-butyl-m-aminophenol used.

### Example 7

207 g (1.0 mole) of N-ethyl-N-isoamylm-aminophenol, 177.6 g (1.2 mole) of phthalic anhydride and 300 g of diphenyl ether ware placed in a reactor, and stirred for 35 hours at 60°C while allowing the resultant keto acid (4-N-ethyl-N-isoamylamino-2-hydroxy-2'-carboxybenzophenone) to deposit in the reaction mixture and the reaction to proceed in a slurry.

The conversion rate of N-ethyl-N-isoamyl-m-aminophenol was 66%: the yield of keto acid (4-N-ethyl-N-isoamylamino-2-hydroxy-2'-carboxybenzophenone) was 65%; and the yield of Rhodamines was 1%.

After the reaction, the reaction mixture was cooled to 30°C so that the keto acid crystalised out, and the crystals were collected by filtration. 1700 ml of a mixture of methanol/water (75/25 in volume ratio) was added to 230 g of the crystals and heated to dissolve the crystals therein. The mixture was the gradually cooled to 20°C to effect recrystallization and the resultant crystals were collected by filtration.

The amount of Rhodamines in the keto acid was found to be not more than 0.1% by liquid chromatographic analysis.

## Claims

1. A method of producing a keto acid of the general formula (I) 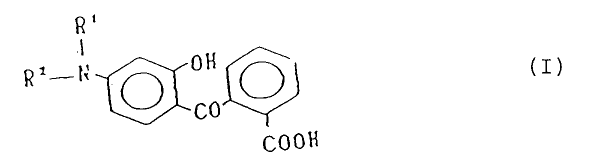 wherein R¹ and R² independently represent alkyl of 1-6 carbons or cycloalkyl of 4-8 carbons, which comprises reacting a m-aminophenol of 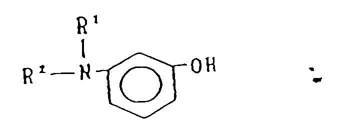 the general formula wherein R¹ and R² are as defined above in relation to formula (I) with phthalic anhydride in the presence of an organic solvent, the organic solvent being present in an amount of 0.5-3 parts by weight per one part by weight of the m-aminophenol with the effect that the resultant keto acid is deposited in the solvent so that the reaction is effected in a slurry.

2. A method according to claim 1 wherein the organic solvent is used in an amount of 0.5-2 parts by weight per part by weight of the m-aminophenol.

3. A method according to claim 1 or 2 wherein the organic solvent is benzene, toluene or xylene.

4. A method according to any one of claims 1 to 3 wherein the organic solvent is diphenyl ether.

5. A method according to any one of claims 1 to 4 which additionally comprises the steps of
(a) cooling the reaction mixture to effect primary crystallization to provide crude crystals of the keto acid;
(b) dissolving the crude crystals in an aliphatic alcohol of 1-4 carbons, or a mixture of the alcohol with water, or a mixture of the alcohol with a hydrocarbon solvent;
(c) effecting secondary crystallization from said alcohol or mixture;
(d) recovering said alcohol or mixture from the resulting crystallization mother liquor; and
(e) adding the recovered keto acid from the mother liquor to the reaction mixture for use in the primary crystallization.

6. A method according to claim 5 wherein in step (a) an aliphatic alcohol of 1-4 carbons is added to the reaction mixture, and then the primary crystallization is effected.

7. A method according to claim 5 or 6 wherein the aliphatic alcohol used in step (a) and/or (b) is methanol or butanol.

8. A method according to any one of claims 5 to 7 wherein the hydrocarbon solvent is an aromatic hydrocarbon of 6-10 carbons or an aliphatic hydrocarbon of 5-10 carbons.

9. A method according to claim 8 wherein the hydrocarbon solvent is toluene, xylene or hexane.

10. A method according to any one of claims 5 to 9 wherein in step (b) the crude crystals are dissolved in said alcohol or mixture under an elevated pressure.

11. A method according to any one of the preceding claims wherein the reaction is effected at a temperature of 60 to 120°C.

## Patentansprüche

1. Verfahren zur Herstellung einer Ketosäure der allgemeinen Formel 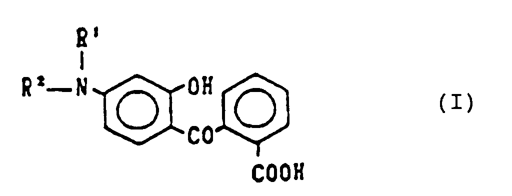 wobei R¹ und R² unabhängig Alkyl mit 1-6 Kohlenstoffatomen oder Cycloalkyl mit 4-8 Kohlenstoffatomen bedeuten, umfassend die Umsetzung eines m-Aminophenols der allgemeinen Formel 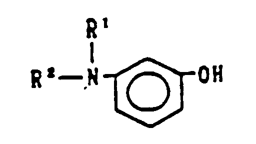 wobei R¹ und R² wie oben in Bezug auf die Formel (I) definiert sind, mit Phthalsäureanhydrid in Gegenwart eines organischen Lösungsmittels, wobei das organische Lösungsmittel in einer Menge von 0,5-3 Gew.-Teilen pro 1 Gew.-Teil des m-Aminophenols vorhanden ist, mit der Wirkung, daß die erhaltene Ketosäure in dem Lösungsmittel abgeschieden wird, so daß die Reaktion in Aufschlämmung durchgeführt wird.

2. Verfahren nach Anspruch 1, wobei das organische Lösungsmittel in einer Menge von 0,5-2 Gewichtsteilen pro Gewichtsteil des m-Aminophenols verwendet wird.

3. Verfahren nach Anspruch 1 oder 2, wobei das organische Lösungsmittel Benzol, Toluol oder Xylol ist.

4. Verfahren nach einem der Ansprüche 1 bis 3, wobei das organische Lösungsmittel Diphenylether ist.

5. Verfahren nach einem der Ansprüche 1 bis 4, umfassend zusätzlich die folgenden Stufen:
(a) Kühlen des Reaktionsgemisches, um eine primäre Kristallisation durchzuführen zur Bildung von rohen Kristallen der Ketosäure;
(b) Lösen der rohen Kristalle in einem aliphatischen Alkohol mit 1-4 Kohlenstoffatomen oder einem Gemisch des Alkohols mit Wasser oder einem Gemisch des Alkohols mit einem Kohlenwasserstoff-Lösungsmittel;
(c) Durchführen einer zweiten Kristallisation aus dem Alkohol oder dem Gemisch;
(d) Gewinnen des Alkohols oder des Gemisches von der erhaltenen Kristallisations-Mutterlauge und
(e) Zugeben der aus der Mutterlauge gewonnenen Ketosäure zu dem Reaktionsgemisch zur Verwendung bei der ersten Kristallisation.

6. Verfahren nach Anspruch 5, wobei in Stufe (a) ein aliphatischer Alkohol mit 1-4 Kohlenstoffatomen zu dem Reaktionsgemisch zugesetzt wird und dann die erste Kristallisation durchgeführt wird.

7. Verfahren nach Anspruch 5 oder 6, wobei der in Stufe (a) und/oder (b) verwendete Alkohol Methanol oder Butanol ist.

8. Verfahren nach einem der Ansprüche 5 bis 7, wobei das Kohlenwasserstoff-Lösungsmittel ein aromatischer Kohlenwasserstoff mit 6-10 Kohlenstoffatomen oder ein aliphatischer Kohlenwasserstoff mit 5-10 Kohlenstoffatomen ist.

9. Verfahren nach Anspruch 8, wobei das Kohlenwasserstoff-Lösungsmittel Toluol, Xylol oder Hexan ist.

10. Verfahren nach einem der Ansprüche 5 bis 9, wobei in Stufe (b) die rohen Kristalle in dem Alkohol oder dem Gemisch unter erhöhtem Druck gelöst werden.

11. Verfahren nach einem der vorangehenden Ansprüche, wobei die Reaktion bei einer Temperatur von 60 bis 120°C durchgeführt wird.

## Revendications

1. Procédé de production d'un cétoacide répondant à la formule générale (I) : 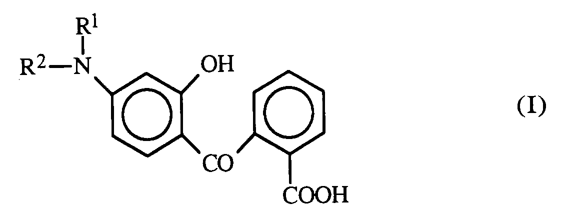 dans laquelle R¹ et R² représentent indépendamment un groupe alkyle comportant de 1 à 6 atomes de carbone ou un groupe cycloalkyle comportant de 4 à 8 atomes de carbone, procédé qui comprend la réaction d'un m-aminophénol de formule générale : 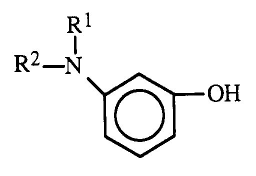 dans laquelle R¹ et R² sont tels que définis précédemment à propos de la formule (I), avec l'anhydride phtalique, en présence d'un solvant organique qui est présent en une quantité de 0,5 à 3 parties en poids pour une partie en poids de m-aminophénol, ce qui a pour effet que le cétoacide résultant précipite dans le solvant de sorte que la réaction s'effectue dans une suspension épaisse.

2. Procédé conforme à la revendication 1, dans lequel le solvant organique est utilisé en une quantité de 0,5 à 2 parties en poids par partie en poids de m-aminophénol.

3. Procédé conforme à la revendication 1 ou 2, dans lequel le solvant organique est le benzène, le toluène ou le xylène.

4. Procédé conforme à l'une quelconque des revendications 1 à 3, dans lequel le solvant organique est l'oxyde de diphényle.

5. Procédé conforme à l'une quelconque des revendications 1 à 4, qui comprend en outre les étapes de :
(a) refroidissement du mélange réactionnel pour effectuer une cristallisation primaire afin d'obtenir des cristaux bruts du cétoacide ;
(b) dissolution des cristaux bruts dans un alcool aliphatique comportant de 1 à 4 atomes de carbone, ou dans un mélange de l'alcool et d'eau, ou dans un mélange de l'alcool et d'un solvant hydrocarboné ;
(c) réalisation d'une cristallisation secondaire à partir dudit alcool ou mélange ;
(d) récupération dudit alcool ou mélange à partir de la liqueur mère de cristallisation résultante ; et
(e) addition du cétoacide récupéré, provenant de la liqueur mère, au mélange réactionnel destiné à être utilisé dans la cristallisation primaire.

6. Procédé conforme à la revendication 5, dans lequel, dans l'étape (a), on ajoute un alcool aliphatique comportant de 1 à 4 atomes de carbone au mélange réactionnel, et puis on effectue la cristallisation primaire.

7. Procédé conforme à la revendication 5 ou 6, dans lequel l'alcool aliphatique utilisé dans l'étape (a) et/ou l'étape (b) est le méthanol ou le butanol.

8. Procédé conforme à l'une quelconque des revendications 5 à 7, dans lequel le solvant hydrocarboné est un hydrocarbure aromatique comportant de 6 à 10 atomes de carbone ou un hydrocarbure aliphatique comportant de 5 à 10 atomes de carbone.

9. Procédé conforme à la revendication 8, dans lequel le solvant hydrocarboné est le toluène, le xylène ou l'hexane.

10. Procédé conforme à l'une quelconque des revendications 5 à 9, dans lequel, dans l'étape (b), on dissout les cristaux bruts dans ledit alcool ou mélange sous une pression élevée.

11. Procédé conforme à l'une quelconque des précédentes revendications, dans lequel on effectue la réaction à une température de 60 à 120° C.
